# EUROPEAN PATENT SPECIFICATION

(11) **EP 2 345 428 B1**
(45) Date of publication and mention of the grant of the patent: **28.09.2016**
(21) Application number: 10153380.0
(22) Date of filing: 11.02.2010
(51) Int. Cl.: A61K 35/761, A61K 48/00, C12N 15/861, A61K 35/76, A61K 38/16, C12N 15/86

(54) **Preparation method of prostate tumor-targeted double-regulated oncolytic adenovirus expressing superantigen gene**
Verfahren zur Herstellung von prostatatumorabgezieltem zweifach reguliertem onkolytischem Adenovirus mit Superantigen-Genexpression
Procédé de préparation d'adénovirus oncolytique régulé doublement ciblé de tumeur de la prostate exprimant un gène super-antigène

(30) Priority: 16.01.2010 CN 201010018143
(43) Date of publication of application: 20.07.2011
(73) Proprietor: Han, Conghui, Quanshan District Xuzhou City Jiangsu (CN)
(72) Inventor: Han, Conghui, Quanshan District Xuzhou City Jiangsu (CN); Hao, Lin, Quanshan District Xuzhou City Jiangsu (CN); Wang, Xiaoliang, 81549 München (DE)
(74) Representative: Beetz & Partner mbB

(56) References cited:
- WO-A2-2005/007832
- US-A- 5 871 726
- DEREK KO ET AL: "Development of transcriptionally regulated oncolytic adenoviruses", ONCOGENE, vol. 24, no. 52, 21 November 2005 (2005-11-21), pages 7763-7774, XP055017513, ISSN: 0950-9232, DOI: 10.1038/sj.onc.1209048
- S SI ET AL: "Gene therapy by membrane-expressed superantigen for [alpha]-fetoprotein-producing hepatocellular carcinoma", GENE THERAPY, vol. 13, no. 22, 1 November 2006 (2006-11-01), pages 1603-1610, XP055017489, ISSN: 0969-7128, DOI: 10.1038/sj.gt.3302823
- HOFFMANN DENNIS ET AL: "Efficient generation of double heterologous promoter controlled oncolytic adenovirus vectors by a single homologous recombination step in Escherichia coli", BMC BIOTECHNOLOGY, vol. 6, no. 1, 3 August 2006 (2006-08-03), pages 36-47, XP021017063, BIOMED CENTRAL LTD. LONDON, GB ISSN: 1472-6750, DOI: 10.1186/1472-6750-6-36
- GUO Z S ET AL: "Oncolytic virotherapy: Molecular targets in tumor-selective replication and carrier cell-mediated delivery of oncolytic viruses", BBA - REVIEWS ON CANCER, vol. 1785, no. 2, 1 April 2008 (2008-04-01), pages 217-231, XP022615368, ELSEVIER SCIENCE BV, AMSTERDAM, NL ISSN: 0304-419X, DOI: 10.1016/J.BBCAN.2008.02.001 [retrieved on 2008-02-15]
- WANG W, JIN B, LI W, XU CX, CUI FA, LIU B, YAN YF, LIU XX, WANG XL.: "Targeted antitumor effect induced by hTERT promoter mediated ODC antisense adenovirus.", MOL BIOL REP., vol. 37, no. 7, 30 October 2009 (2009-10-30), pages 3239-3247, XP002667866,
- YUURI HASHIMOTO ET AL: "Establishment of biological and pharmacokinetic assays of telomerase-specific replication-selective adenovirus", CANCER SCIENCE, vol. 99, no. 2, 1 February 2008 (2008-02-01), pages 385-390, XP055017407, ISSN: 1347-9032, DOI: 10.1111/j.1349-7006.2007.00665.x
- Z-B HU ET AL: "A simplified system for generating oncolytic adenovirus vector carrying one or two transgenes", CANCER GENE THERAPY, vol. 15, no. 3, 1 March 2008 (2008-03-01), pages 173-182, XP055017566, ISSN: 0929-1903, DOI: 10.1038/sj.cgt.7701105
- P HUANG ET AL: "Direct and distant antitumor effects of a telomerase-selective oncolytic adenoviral agent, OBP-301, in a mouse prostate cancer model", CANCER GENE THERAPY, vol. 15, no. 5, 1 May 2008 (2008-05-01), pages 315-322, XP055017569, ISSN: 0929-1903, DOI: 10.1038/cgt.2008.3
- JOHN IRVING ET AL: "Conditionally replicative adenovirus driven by the human telomerase promoter provides broad-spectrum antitumor activity without liver toxicity", CANCER GENE THERAPY, vol. 11, no. 3, 1 March 2004 (2004-03-01), pages 174-185, XP055017570, ISSN: 0929-1903, DOI: 10.1038/sj.cgt.7700666
- SMALL ET AL: "A Phase I Trial of Intravenous CG7870, a Replication-Selective, Prostate-Specific Antigen-Targeted Oncolytic Adenovirus, for the Treatment of Hormone-Refractory, Metastatic Prostate Cancer", MOLECULAR THERAPY, vol. 14, no. 1, 1 July 2006 (2006-07-01), pages 107-117, XP005560377, ACADEMIC PRESS, SAN DIEGO, CA, US ISSN: 1525-0016

## Description

### BACKGROUND OF THE INVENTION

### Field of the Invention

The present invention relates to prostate tumor targeted expression of a superantigen gene, in particularly to a preparation method of a prostate tumor-targeted double-regulated oncolytic adenovirus expressing a superantigen gene, and belongs to the technical field of gene therapy.

### Related Art

Prostate cancer is one of the most common malignant tumors in the male reproductive system, and in recent years, its incidence is increasing year by year. A large amount of survey data indicates that, in China, the prostate cancer incidence and related deaths are increasing year by year, and the diagnosis and treatment of prostate cancer have become an important research content of modern medicine. Presently, there are many strategies for prostate cancer gene therapy. As most of the therapies do not have good specificity, and cannot directly target prostate cancer cells, thus being limited in clinical application. As we all know, the body's immune response has anti-tumor effect, by capturing and eliminating tumor cells newly grown in the body through cell immune function. The final factor that causes oncogenesis can be attributed to the quantity and quality of immune effector cells insufficient to stimulate an effective anti-tumor immune response. Therefore, in terms of therapy, effective anti-tumor strategies include: (1) enhancing the body's anti-tumor immune response, mainly by increasing the number of locally-activated immune effect cells in tumor, with major histocompatibility complex (MHC) restricted cytotoxic T lymphocytes (CTL, i.e., killer T cells) being the most important immune surveillance and effector cells; and (2) taking effective measures to directly kill tumor cells.

Super-antigens are a group of protein molecules encoded by bacteria or viruses, and can bind as a whole protein molecule directly to the outside of the antigen binding groove of MHC-II class molecules on the membrane of the antigen-presenting cell (APC) and be presented to T cells, without need of the presenting effect of APC. The superantigen-MHC-II class molecule complex merely binds to the V region of the T cell antigen receptor β chain. As there are more than 20 human V_{β} genes, the clones of T cells activated by superantigens can be a few thousands to tens of thousands times more than that of common antigens. The anti-tumor effect of superantigens is mainly achieved by the superantigen (SAg) dependent cell mediated cytotoxicity, which is the main effect of superantigens against the tumor. Furthermore, CD4⁺T cells activated by superantigens can secrete a variety of cytokines in sufficient quantity, such as, tumor necrosis factor-a, tumor necrosis factor-β, and tumor necrosis factor-y, interleukin-2, and interleukin-6, to directly or indirectly kill tumor cells. Research on superantigens, as a new anti-tumor therapy mode, has developed rapidly in recent years, which is mainly focused on the direct anti-tumor effect of superantigens, targeting anti-tumor effect of superantigens, and superantigen gene therapy, and so on. Staphylococcal enterotoxin A (SEA) is the most studied superantigen currently.

S. Si et al. describe for example in "Gene therapy by membrane-expressed superantigen for α-fetoprotein-producing hepatocellular carcinoma", Gene Therapy (2006) 13, 1603-1610, the use of staphylococcus enterotoxin A (SEA) for treating hepatocellular carcinoma.

However, there are still many difficulties to be overcome in either anti-tumor therapy with superantigens alone or currently antibody targeted superantigen anti-tumor therapy. As for therapy with superantigens alone, an obvious disadvantage is in that, superantigen activated killer T cells mainly kill MHC-II-positive tumor cells, and have a weak cytotoxic effect on MHC-II-negative tumor cells. As tumor cells generally have a low MHC-II-positive rate and have significant heterogeneity, the anti-tumor effect of simple superantigens is not desirable, and the toxicity on the body's MHC-II positive normal cells occurs inevitably. Further, T cells activated by monoclonal antibody-superantigen fusion proteins are SEA reactive, and are not tumor specific. Therefore, such superantigen-monoclonal antibody fusion proteins lack broad-spectrum activity and specificity against the tumor, and targeting immune responses to the tumor induced thereby is not satisfactory.

The most critical measure for gene therapy is to select a suitable vector with a high efficiency to deliver the gene into the target organ. Presently, virus vectors are still dominant in the research of vectors for tumor gene therapy. Oncolytic adenovirus, obtained by modifying a known adenovirus, has specific killing effect on the defects of tumor gene genotype, and functions as a targeting vector for anti-tumor drugs. As the killing effect of oncolytic adenovirus on tumor cells is specific and safe, oncolytic adenovirus has become a new technology of biological treatment of tumor with promising prospect. During self-amplification, tumor cells will generate some abnormally expressed products with respect to normal cells, which products can stimulate the expression of some genes, thus stimulating the amplification of cells or viruses. The gene fragments that can bind to the tumor expressed abnormal molecules and initiate downstream gene expression are referred to as tumor-specific promoters. In the construction of oncolytic adenovirus, the modification of the genome of the adenovirus by the tumor-specific promoters is one of the hot issues in current research. The adenovirus replication is regulated by tumor specific enhancers, so as to enhance the viral infection ability on tumor cells.

Telomerase is in an activated state in most malignant tumor cells, and has no activity or low activity in normal cells, and is a tumor bimolecular marker having the most broad-spectrum activity that has been reported presently. Human telomerase reverse transcriptase (hTERT) is the catalytic subunit required for maintaining the activity of telomerase, and the expression of hTERT is regulated at transcriptional level. Theoretically, regulation of the gene required for virus replication and proliferation by the telomerase reverse transcriptase promoter can result in the selective proliferation of the virus in telomerase-positive tumor cells. If the promoter of the E1A gene itself is replaced by the telomerase reverse transcriptase promoter, the expression of the E1A gene can be restricted to the tumor cells, and there is no expression in normal cells.

In recent years, the prostate cancer specific molecular marker-prostate specific antigen has become one of the hot issues in prostate cancer clinical research. The expression of prostate specific antigen is closely related to prostate cancer, and has a more and more important effect in the early diagnosis, gene therapy, and prognosis of prostate cancer. The determination of serum prostate specific antigen is of important significance in screening, early diagnosis, and disease prediction of prostate cancer, and has been widely used in clinics and become preferred test item in guideline for diagnosis and treatment of PCa in China. Prostate specific antigen is a specific single-chain protein secreted by prostate epithelial cells in human prostate tissue. As the prostate specific antigen is almost expressed merely in prostate cancer tissue, the promoter of the gene has high tissue specificity. Therefore, construction of a tissue specific vector with a regulation sequence for transcription of the protein enables the therapy gene to be selectively expressed in prostate cancer, to achieve the purpose of specifically killing prostate tumor cells.

Treating tumor with oncolytic adenovirus, as a new method for tumor treatment, has inherent unique advantages, but still has some problems to be solved, which mainly includes the following aspects.

Firstly, the specific killing capacity on the tumor needs to be enhanced. Presently, through the treatment on adenovirus E1B, the targeting oncolysis on the tumor cells of p53 defects can be achieved. Research results show that a part of early tumor cells has low heteromorphism with respect to normal cells and weak adenovirus effect, so the treatment of this part of cells has to rely on the combined radiotherapy and chemotherapy to kill the tumor, thus the oncolytic adenovirus has weak specific killing on the part of tumor cells.

Secondly, as the adenovirus for oncolysis is introduced into the body as a foreign material, the body will definitely generate defensive effect against the virus, thus fever, injection site reactions, flu-like symptoms, leukopenia, thrombocytopenia, liver and kidney dysfunction, hair loss, nausea and vomiting, and other adverse reactions may occur. The emergence of the adverse reactions indicates that the body's own immune response has an effect on the virus, thus the viral titer is reduced to some extent, and the killing effect of the virus on tumor cells will, of course, be reduced.

Additionally, the first step of the virus acting on the tumor cells is to specifically affect some immune molecules on the surface of tumor cells, but tumor cells sometimes does not express such molecules, and cannot be infected by the virus. The application in tumor gene therapy is limited by natural properties of C group adenoviruses, partially due to low expression level of the C group adenovirus receptor in the malignant tumor.

Derek Ko. et al. summarize in "Development of transcriptionally regulated oncolytic adenoviruses", Oncogene (2005) 24, 7763-7774, different approaches for the use of replicating viruses to treat tumors.

### SUMMARY OF THE INVENTION

Accordingly, in order to overcome the disadvantages in prior art, the present invention is directed to a preparation method of a prostate tumor-targeted double-regulated oncolytic adenovirus expressing a superantigen gene. According to the method, using the gene engineering technology, a superantigen SEA gene is cloned into an oncolytic adenovirus vector to limit its proliferation and expression merely in prostate tumor cells, so as to achieve the purpose of targeting and killing prostate tumor cells; and the tumor-specific replicative adenovirus vector is regulated by a prostate-specific antigen promoter (PSAP) and the telomerase reverse transcriptase promoter, to carry a SEA gene fragment , which has both specific proliferation capacity in tumor cells and strong cytotoxic T lymphocyte (CTL) stimulating capacity.

The present invention is realized by the following technical solution.

A prostate tumor-targeted double-regulated oncolytic adenovirus expressing a superantigen gene is provided, which is characterized in that it includes an oncolytic adenovirus part and a superantigen part. The oncolytic adenovirus part includes an oncolytic adenovirus shuttle plasmid, and a telomerase reverse transcriptase gene promoter and a PSAP for regulating the oncolytic adenovirus. The superantigen part is a SEA gene fragment.

The SEA gene fragment has a length of 771bp.

The dual-promoter for regulating the oncolytic adenovirus is a telomerase reverse transcriptase gene promoter and a PSAP.

As for the dual-promoter, the telomerase reverse transcriptase gene promoter is carried by the virus vector itself, and the PSAP is extracted from prostate cancer tissues, and according to sequence U37672.1 from US NCBI Nucleotide, upstream and downstream primers are designed to amplify the PSAP, and a PSAP fragment having a size of 522bp is amplified.

A preparation method of a prostate tumor-targeted double-regulated oncolytic adenovirus expressing a superantigen gene is provided, which includes:
(1) cloning a PSAP;
(2) constructing an oncolytic adenovirus vector, including cloning the PSAP into an oncolytic adenovirus shuttle plasmid comprising a telomerase reverse transcriptase gene promoter to get a virus plasmid comprising PSAP, and recombining it with a SEA gene-carrying plasmid PPE3-ccdb-SEA; and
(3) performing packaging, purification, and titer determination on the oncolytic adenovirus.

The present invention has the following advantages. (1) According to two main features of solid tumor of unlimited proliferation and of tumor hypoxia, as E1A and E1B promoters necessary for adenovirus vector replication are replaced by the telomerase reverse transcriptase promoter and the PSAP respectively, such that the proliferation of oncolytic adenovirus vector can be more effectively controlled in the prostate tumor cells merely, thus achieving the purpose of targeting delivery of the SEA gene with a high efficiency. (2) As therapy gene, the SEA gene is tumor targeted, and can be locally replicated in tumor with a high efficiency, to stimulate a strong anti-tumor immune response, thus significantly increasing the tumor killing effect and avoiding the side-effects of the superantigen in systematic application as maximum as possible. (3) As vector, the tumor specific replicative adenovirus can infect tumor cells specifically and be replicated and proliferated therein, to lyse and kill tumor cells and release more viruses, so as to infect other tumor cells around for a greater range of lysis and killing to form a chain reaction.

Further scope of applicability of the present invention will become apparent from the detailed description given hereinafter. However, it should be understood that the detailed description and specific examples, while indicating preferred embodiments of the invention, are given by way of illustration only, since various changes and modifications within the spirit and scope of the invention will become apparent to those skilled in the art from this detailed description.

### BRIEF DESCRIPTION OF THE DRAWINGS

The present invention will become more fully understood from the detailed description given herein below for illustration only, and thus are not limitative of the present invention, and wherein:
FIG. 1 is the electrophoresis result of pUC57-PSAp plasmid after Notl and Spel double-digestion;
FIG. 2 is the sequence of prostate specific antigen promoter (522bp);

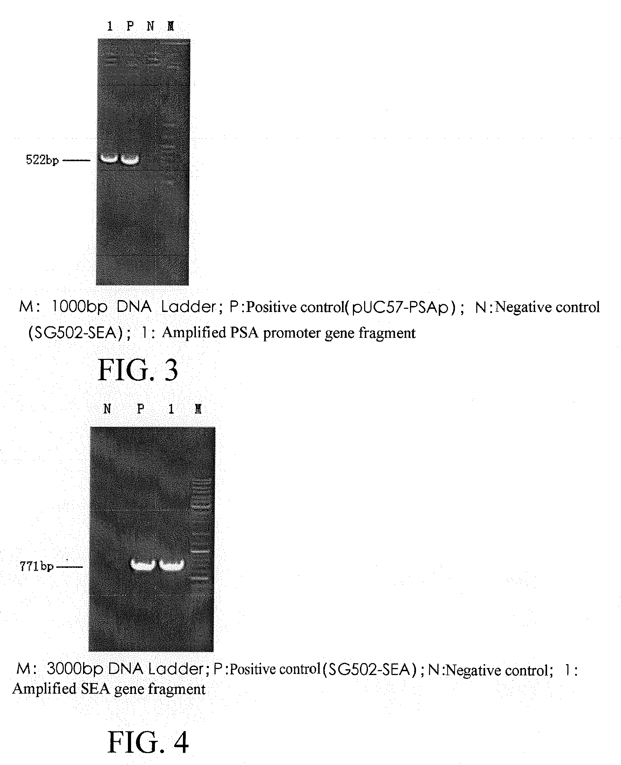

FIG. 3 is the electrophoresis result of SG502-SEA after PCR amplification with PSAp primer;
FIG. 4 is the electrophoresis result of SG504-SEA after PCR amplification with SEA primer; and

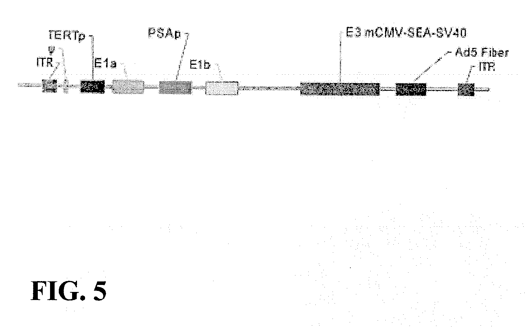

FIG. 5 is a structural view of constructed superantigen SEA gene-carrying double-regulated replicative adenovirus SG504-SEA.

### DETAILED DESCRIPTION OF THE INVENTION

### Embodiments

### 1 Reagents for experiment

Virus vector pSG502 and HEK293 cell were purchased from the Virus and Gene Therapy Center of Shanghai Hepatobiliary Surgery Hospital. The SEA gene-carrying viral backbone plasmid PPE3-ccdb-SEA was stored in this laboratory.

| | |
|---|---|
| pUC57 cloning vector | Sangon Company |
| Taq enzyme | Shenergy Biocolo Company |
| DNA ligase Solution I | TakaRa Company |
| Protease K, RNase | PROMEGA Company |
| Fetal bovine serum (FBS), bovine calf serum (BCF)DMEM | GIBCO BRL Company |
| Agarose, ethidium bromide (EtBr) | GENE Company |
| Agar powder, tryptone, yeast extract | UNIPATH Company |
| Gel extraction kit | QIAGEN Company |
| PCR product extraction kit | QIAGEN Company |
| Plasmid DNA preparation kit | QIAGEN Company |
| Virus DNA extraction kit | QIAGEN Company |
| LipofectAmine2000 kit | GIBCO BRL Company |

### 2. Cloning of prostate specific promoter

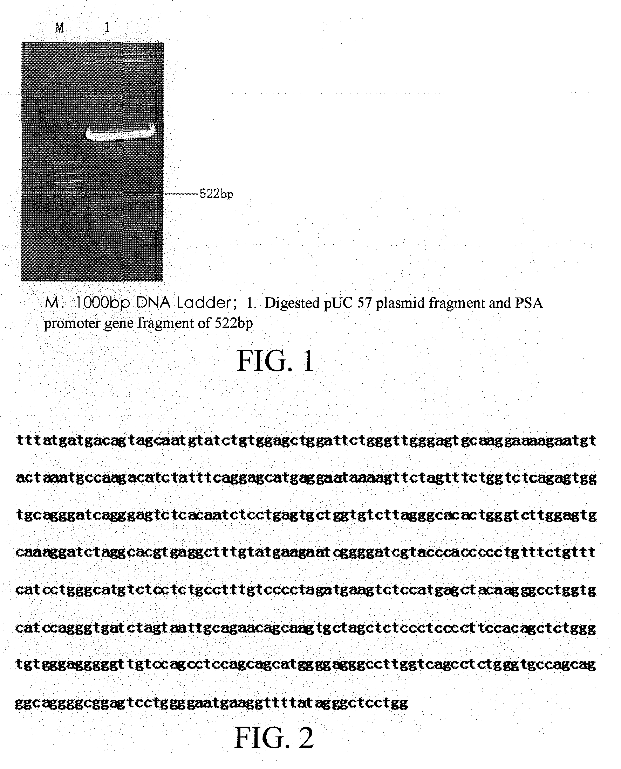

According to sequence (U37672.1) from US NCBI Nucleotide, upstream primer P: 5'-atatgcggccgctttatgatgacagtag-3' and downstream primer P: 5'-gtgtactagtccaggagccctataaa-3' were designed to amplify the PSA promoter, and introduced into NotI and SpeI digestion sites, respectively. The genome DNA was extracted from the prostate cancer tissue, and was used as template for PCR amplification to get a PSA promoter fragment of 522bp. Cycle parameters included: pre-degeneration at 94°C for 4 min, degeneration at 94°C for 30 s, annealing at 56°C for 1 min, extension at 72°C for 1 min, for 35 cycles, and finally, extension at 72°C for 7 min; the amplification product was identified with 1% agarose gel electrophoresis. Through the EcoR V digested pUC57 plasmid, the PCR product of the target gene was ligated to the pUC57 vector digestion product, and reacted at 16°C overnight. The JM109 competent cells were transformed and plated on an ampicillin resistant plate, cultured at 37°C overnight, and screened for clones. The plasmid was extracted and sequenced to determine the insertion product. The plasmid identified to be correct was named as pUC57-PSAp, as shown in FIG. 1.

### 3. Construction and recombination of superantigen SEA gene-carrying hTERT/PSA double-regulated replicative oncolytic adenovirus SG502-SEA

### 3.1. Digestion and extraction

| | | | | |
|---|---|---|---|---|
| PUC57-PSAp | 10.0 µl | | SG502 | 2.0 µl |
| Spe I | 3.0 µl | | Spe I | 3.0 µl |
| Not I | 3.0 µl | | Not I | 3.0 µ1 |
| NEB 2 | 10.0 µl | | NEB 2 | 8.0 µl |
| 10*BSA | 10.0 µl | | 10*BSA | 8.0 µl |
| H₂O | 64.0 ul | | H₂O | 56.0 ul |
| Total system | 100.0 µl | | Total system | 80.0 µl |

After water bath at 37°C for 6 h, 1.2% agarose gel electrophoresis was performed, and PUC57-PSAp of 522 bp, and SG502 of 10087 bp were extracted using a gel extraction kit (QIAquick Gel Extraction).

### 3.2. Ligation

| | |
|---|---|
| PUC57-PSAp / Spe I+Not I | 8 µl |
| SG502/ Spe I+Not I | 2 µl |
| Solution I | 10 µl |
| Total system | 20.0 µl |

After ligation at 12°C for 12 h, the ligation product was transformed into DH5α *E. coli* competent cells. The cells were plated on an agar plate (containing AMP), and cultured at 37°C in a biochemical incubator for 10-12 h. The grown single bacterial clones was picked up and amplified in a LB solution containing AMP in a shaker at 37°C for 12 h. The plasmid DNA of positive clones was extracted, and was named as SG504 after being identified to be correct by digestion.

### 3.3 Recombination and identification

The plasmid PPE3-ccdb-SEA and SG504 were co-transfected into 293 cells with Lipofectamine2000. 9-14 days after co-transfection, viral plaques appeared, and after 3 times of viral plaque purification, the adenovirus DNA was extracted with QIAGEN DNA Blood Mini Kit (QIAGEN Company) and identified by PCR. The adenovirus identified to be correct was named as SG504-SEA, that is, SEA gene-carrying regulated tumor specific replicative adenovirus (see FIG. 2).

### 3.31 Culture of 293 cells

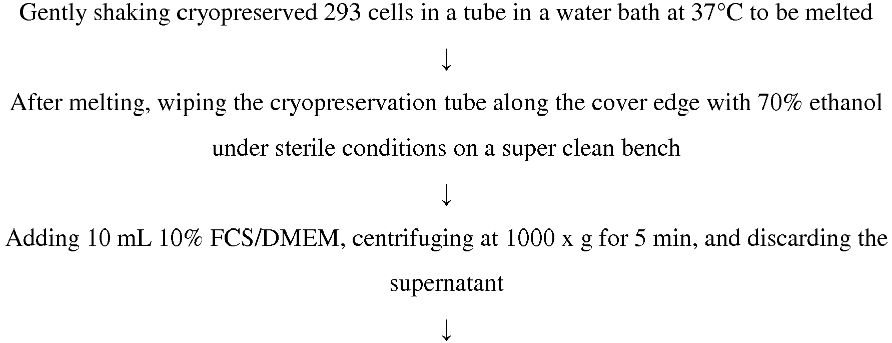

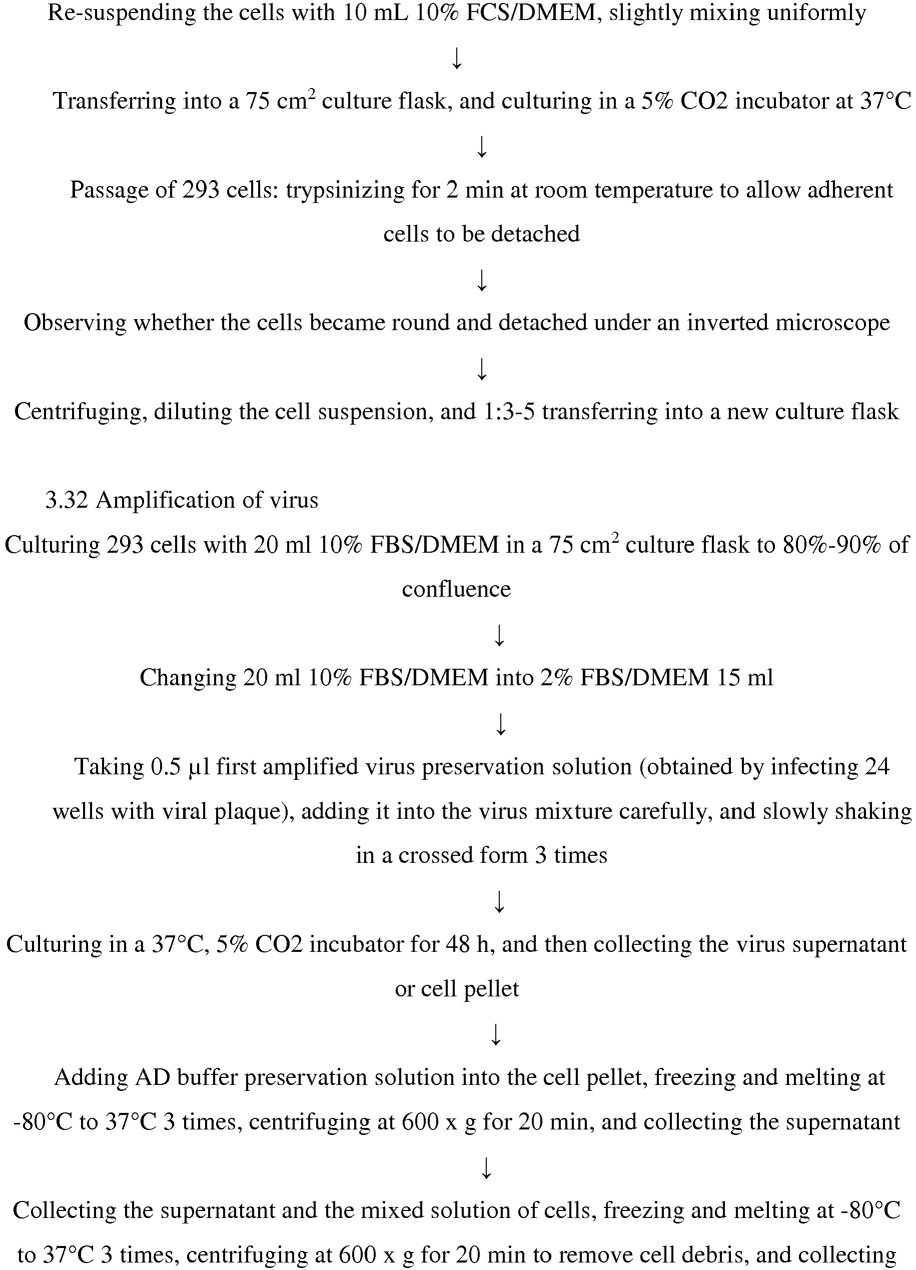

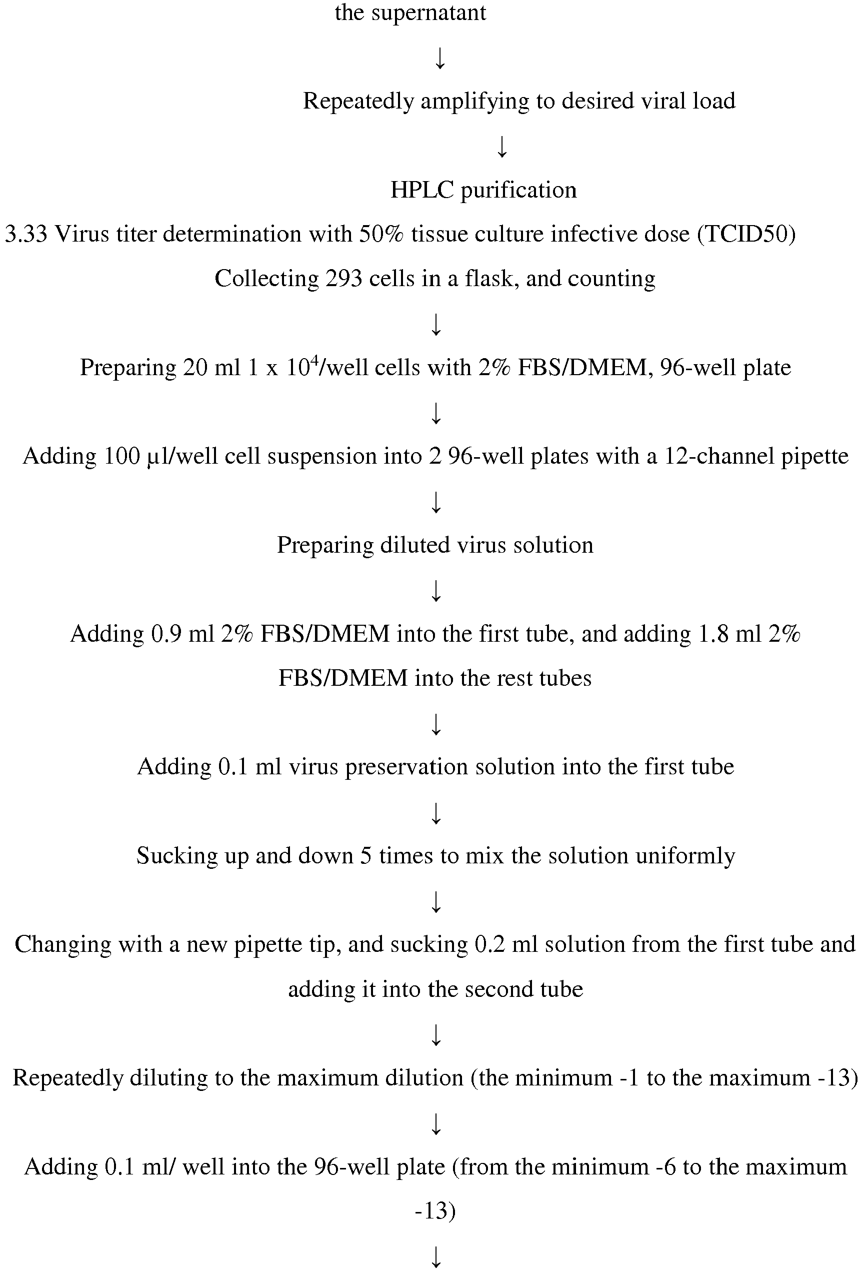

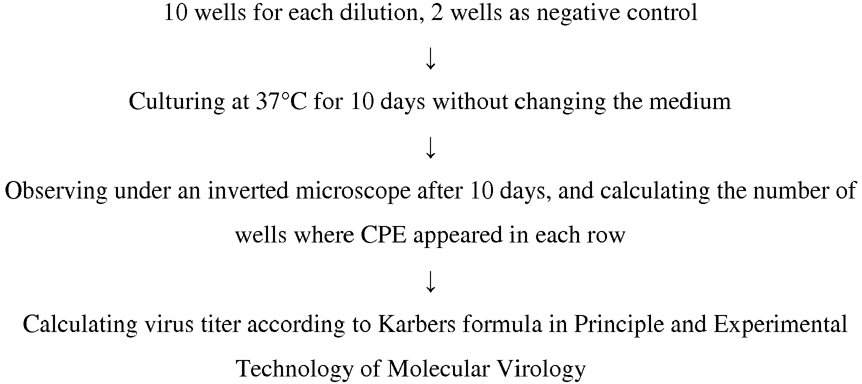

### 4 Amplification and titer determination of superantigen SEA gene-carrying hTERT/PSA double-regulated replicative oncolytic adenovirus SG504-SEA

After 293 cells were cultured with 20 ml 10% FBS/DMEM in a 75 cm² culture flask to 80%-90% of confluence, 20 ml 10% FBS/DMEM was changed into 15 ml 2% FBS/DMEM, and 0.5 µl first amplified virus preservation solution (obtained by infecting 24 wells with viral plaque) was taken, the virus mixture was added carefully, and shaken slowly in a cross form 3 times. After culturing in a 37°C, 5% CO2 incubator for 48 h, the virus supernatant or cell pellet was collected. Into the precipitated cells, AD buffer preservation solution was added, and frozen and melted at -80°C to 37°C 3 times, centrifuged at 600 x g for 20 min, the precipitate was discarded and the supernatant was taken. The supernatant and the mixed solution of cells were collected, and frozen and melted at -80°C to 37°C 3 times, centrifuged at 600 x g for 20 min to remove cell debris, and the supernatant was collected. Amplification was repeated to the desired viral load, and the virus titer was determined with 50% tissue culture infective dose (TCID50).

## Claims

1. A prostate tumor-targeted double-regulated oncolytic adenovirus expressing a superantigen gene, comprising an oncolytic adenovirus part and a superantigen part, wherein the oncolytic adenovirus part comprises an oncolytic adenovirus shuttle plasmid and a telomerase reverse transcriptase gene (TERT) promoter and a prostate-specific antigen promoter (PSAP) for regulating the oncolytic adenovirus, and the superantigen part is a staphylococcal enterotoxin A (SEA) gene fragment.

2. The prostate tumor-targeted double-regulated oncolytic adenovirus expressing a superantigen gene according to claim 1, wherein the SEA gene fragment has a length of 771bp.

3. The prostate tumor-targeted double-regulated oncolytic adenovirus expressing a superantigen gene according to claim 1 or 2, wherein the dual-promoter for regulating the oncolytic adenovirus is a telomerase reverse transcriptase gene promoter and a PSAP.

4. A preparation method of a prostate tumor-targeted double-regulated oncolytic adenovirus expressing a superantigen gene according to claim 1, comprising:
(1) cloning a prostate-specific antigen promoter (PSAP);
(2) constructing an oncolytic adenovirus vector, comprising cloning the PSAP into an oncolytic adenovirus shuttle plasmid comprising a telomerase reverse transcriptase gene promoter to get a virus plasmid comprising PSAP, and recombining it with a SEA gene-carrying plasmid PPE3-ccdb-SEA; and
(3) performing packaging, purification, and titer determination on the oncolytic adenovirus.

## Patentansprüche

1. Doppelt reguliertes, auf einen Prostatatumor gerichtetes, onkolytisches Adenovirus, das ein Superantigen-Gen exprimiert, mit einem onkolytischen Adenovirusteil und einem Superantigenteil, wobei der onkolytische Adenovirusteil ein onkolytisches Adenovirus-Shuttle-Plasmid und einen Telomerase-Reverse-Transcriptase-Gen (TERT)-Promotor und einen prostataspezifisches Antigen-Promotor (PSAP) zur Regulation des onkolytischen Adenovirus aufweist und wobei es sich bei dem Superantigenteil um ein Staphylokokkenenterotoxin A (SEA)-Genfragment handelt.

2. Doppelt reguliertes, auf einen Prostatatumor gerichtetes, onkolytisches Adenovirus, das ein Superantigen-Gen exprimiert, nach Anspruch 1, wobei das SEA-Fragment eine Länge von 771 bp aufweist.

3. Doppelt reguliertes, auf einen Prostatatumor gerichtetes, onkolytisches Adenovirus, das ein Superantigen-Gen exprimiert, nach Anspruch 1 oder 2, wobei es sich bei dem Doppelpromotor zur Regulation des onkolytischen Adenovirus um einen Telomerase-Reverse-Transcriptase-Gene-Promotor und einen PSAP handelt.

4. Verfahren zur Herstellung eines doppelt regulierten, auf einen Prostatatumor gerichteten, onkolytischen Adenovirus, das ein Superantigen-Gen exprimiert, nach Anspruch 1, wobei das Verfahren umfasst:
(1) Klonieren eines prostataspezifischen Antigen-Promotors (PSAP) ;
(2) Konstruieren eines onkolytischen Adenovirusvektors, das umfasst, den PSAP in ein onkolytisches Adenovirus-Shuttle-Plasmid zu klonieren, das einen Telomerase-Reverse-Transcriptase-Gen (TERT)-Promotor aufweist, sodass ein Virusplasmid mit einem PSAP erhalten wird, und Rekombinieren des Plasmids mit einem SEA-Gen-enthaltenden Plasmid PPE3-ccdb-SEA; und
(3) Konfektionieren, Reinigen und Bestimmen des Titers an dem onkolytischen Adenovirus.

## Revendications

1. Adénovirus oncolytique doublement régulé ciblé pour une tumeur de la prostate qui exprime un gène codant un superantigène, qui comprend une portion adénovirus oncolytique et une portion superantigène, où la portion adénovirus oncolytique comprend un plasmide navette contenant l'adénovirus oncolytique et un promoteur du gène codant le composant à activité transcriptase inverse de la télomérase (TERT) et un promoteur de l'antigène spécifique de la prostate (PSAP) assurant la régulation de l'adénovirus oncolytique, et où la portion superantigène est un fragment du gène codant l'entérotoxine A staphylococcique (SEA).

2. Adénovirus oncolytique doublement régulé ciblé pour une tumeur de la prostate qui exprime un gène codant un superantigène selon la revendication 1, où le fragment du gène codant la SEA a une longueur de 771 paires de bases.

3. Adénovirus oncolytique doublement régulé ciblé pour une tumeur de la prostate qui exprime un gène codant un superantigène selon la revendication 1 ou 2, où le double promoteur assurant la régulation de l'adénovirus oncolytique est un promoteur du gène codant le composant à activité transcriptase inverse de la télomérase et un PSAP.

4. Procédé de préparation d'un adénovirus oncolytique doublement régulé ciblé pour une tumeur de la prostate qui exprime un gène codant un superantigène selon la revendication 1, qui comprend :
(1) le clonage d'un promoteur de l'antigène spécifique de la prostate (PSAP) ;
(2) la construction d'un vecteur incluant l'adénovirus oncolytique, qui fait intervenir le clonage du PSAP dans un plasmide navette contenant l'adénovirus oncolytique qui comprend un promoteur du gène codant le composant à activité transcriptase inverse de la télomérase pour obtenir un plasmide viral comprenant le PSAP et sa recombinaison avec un plasmide portant le gène de la SEA (PPE3-ccdb-SEA) ; et
(3) la réalisation de l'empaquetage, de la purification et de la détermination du titre de l'adénovirus oncolytique.
